(19) Europäisches Patentamt
European Patent Office
Office européen des brevets

(11) **EP 1 885 310 B1**

(12) **FASCICULE DE BREVET EUROPEEN**

(45) Date de publication et mention
de la délivrance du brevet:
**12.03.2014 Bulletin 2014/11**

(21) Numéro de dépôt: **06755460.0**

(22) Date de dépôt: **28.04.2006**

(51) Int Cl.:
***A61F 13/08*** *(2006.01)*

(86) Numéro de dépôt international:
**PCT/FR2006/000956**

(87) Numéro de publication internationale:
**WO 2006/117459 (09.11.2006 Gazette 2006/45)**

(54) **ORTHESE COMPRESSIVE DE CONTENTION DU MEMBRE INFÉRIEUR EN FORME D'ARTICLE TRICOTÉ DE TYPE BAS, CHAUSSETTE OU COLLANT**

KOMPRESSIONSORTHESE FÜR DIE UNTEREN GLIEDMASSEN IN FORM EINES STRUMPFES, EINER SOCKE ODER EINES ALS STRUMPFHOSE GEFORMTEN GESTRICKTEN ARTIKELS

LOWER LIMB SETTING COMPRESSION ORTHESIS IN THE FORM OF A STOCKING, SOCK OR PANTY HOSE-SHAPED KNITTED ARTICLE

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **02.05.2005 FR 0504436**

(43) Date de publication de la demande:
**13.02.2008 Bulletin 2008/07**

(73) Titulaire: **Innothera Topic International S.A.
94111 Arcueil (FR)**

(72) Inventeurs:
• **GOBET, Arnaud
F-75007 Paris (FR)**
• **CROS, François
F-94200 Ivry Sur Seine (FR)**

(74) Mandataire: **Dupuis-Latour, Dominique et al
Bardehle Pagenberg
10, boulevard Haussmann
75009 Paris (FR)**

(56) Documents cités:
**EP-A- 0 705 543     US-A- 3 856 008
US-A- 4 527 402**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

L'invention concerne les orthèses généralement connues sous la dénomination de "bas de contention" ou "collants de contention", destinées au traitement de l'insuffisance veineuse chronique (IVC).

Bien que dans la suite on utilisera le terme "bas", invention n'est toutefois pas limitée à un type d'article particulier, mais s'applique aussi bien à toutes les formes d'orthèses compressives d'un ou des deux membres inférieurs, qu'il s'agisse de collants, de mono-collants, de bas ou de chaussettes.

L'invention concerne spécifiquement les "bas de contention", qui sont des bas médicaux produisant un effet thérapeutique par compression/contention des membres inférieurs, par opposition aux "bas de maintien" (ou encore "bas de soutien" ou "bas anti-fatigue") et aux "bas mode", qui ne sont pas des bas médicaux à visée thérapeutique.

Ces bas de contention médicaux sont des bas qui exercent une pression, mesurée à la cheville, de 10 à plus de 36 mmHg (13 à 48 hPa ; on utilisera toutefois dans la présente description le mmHg comme unité de mesure de pression compte tenu de son usage universel dans le domaine de la phlébologie et de la contention médicale). Ces bas sont répartis selon la norme française en quatre classes de contention, à savoir la classe I (10 à 15 mmHg à la cheville), la classe II (15 à 20 mmHg), la classe III (20 à 36 mmHg) et la classe IV (> 36 mmHg).

On notera que ces bas de contention destinés à traiter l'insuffisance veineuse chronique relèvent d'un domaine thérapeutique tout à fait différent de celui des orthèses telles que celle décrite par exemple dans le US-A-3 856 008, qui appliquent à la jambe des pressions très élevées, pouvant aller jusqu'à 150 mmHg à la cheville, afin de traiter des cas de brûlures de la jambe ou de lymphoedème.

Le EP 0 934 043 B1 décrit un article de contention destiné aux sportifs, avec un gradient de pressions particulier, opposé aux bas de contention des insuffisants veineux. Cet article comprend une première partie enveloppant le pied et les malléoles, et une seconde partie enveloppant le mollet, exerçant une pression moyenne inférieure à celle exercée par la première partie. Aucune indication n'est toutefois donnée sur la manière dont varie la pression au-dessous des malléoles, au niveau de la seconde partie.

Les bas de contention sont réalisés en un matériau élastique, typiquement une maille tricotée de texture très serrée avec, en outre, incorporation d'un fil de trame élastique (généralement un élasthanne guipé), Ils sont dimensionnés en fonction de la jambe du patient, pour obtenir par élasticité le degré et le profil de pression recherchés.

Le point de départ de l'invention est la constatation du fait que de nombreux utilisateurs de ces bas de contention se plaignent de sensations d'inconfort au niveau du cou de pied, et de refroidissement significatif du pied après quelques heures de port. Ce phénomène est dû à la texture très serrée des bas de contention, qui est nécessaire pour produire un effet médical sur la jambe mais entraîne sur le pied, notamment au niveau des régions malléolaire et du cou de pied, une compression résiduelle produisant une excès de pression entraînant les inconvénients évoqués ci-dessus. Ces inconvénients n'apparaissent pas avec les bas de maintien et les bas mode, qui n'exercent qu'une pression beaucoup plus faible.

Ce phénomène est d'autant plus marqué avec les bas de contention que, compte tenu du profil de pression dégressive, c'est au niveau de la cheville que le bas exerce la pression la plus élevée, donc à proximité de l'articulation du pied, c'est-à-dire là où il n'est médicalement pas nécessaire d'exercer une pression trop forte (par "pied" on entendra toute la région du membre inférieur allant de la région des malléoles - les crêtes osseuses tibiale et péronéale - jusqu'à l'extrémité des orteils, le "cou de pied" étant défini comme la partie supérieure antérieure située dans la région de la courbure du pied).

Or la compression au niveau du pied n'a pas d'intérêt démontré et ne semble guère réaliste, dans la mesuré où :

- l'anatomie de l'arche plantaire s'y prête peu et les veines à comprimer éventuellement (la semelle plantaire) sont sous l'arche. En conséquence de la loi de Laplace, la compression a toute chance d'être d'abord vulnérable au niveau du cou de pied et des bords interne et externe de la plante du pied (1$^{er}$ et 5$^e$ rayon) avant d'être éventuellement, et tout au plus faiblement, efficace sur le réseau veineux de la semelle plantaire ("semelle de Lejars"). En fait, pour être efficace il faudrait mettre une orthèse moulée sur la forme de l'arche plantaire (ou n'attendre une efficacité que chez les sujets aux pieds plats grade 3).

- en position couchée, la position naturelle du pied est favorable au drainage veineux de l'avant-pied, et en position debout l'écrasement de la semelle plantaire fait, en termes de compression, sans doute mieux que ce que ferait un bas très compressif au niveau du pied. La seule position vulnérable est la position assise avec inactivité totale du pied et pied et de la cheville.

De plus il existe des limites mineures, ou des réserves franches, à la compression au niveau du pied :

- chez certains sujets, la vascularisation artérielle de l'avant-pied est vulnérable du fait de l'anatomie elle-même. Normalement, cette vascularisation bénéficie de l'anastomose entre les réseaux de l'artère pédieuse (branche terminale de la tibiale antérieure, qui court sur le cou de pied et le dos du pied avant de plonger entre les métatarsiens) et de la plantaire interne (branche de l'artère tibiale postérieure, qui court au bord interne de l'arche plantaire). Quelquefois cette anastomose n'existe pas, et la vascularisation artérielle des orteils s'en trouve fragilisée. Ceci est sans doute responsable de la sensation d'avant-pied froid ressentie par certains patients.

- chez d'autres patients, ce seront les déformations de l'avant-pied (hallux valgus, quintus varus, avant-pied rond) qui poseront problème, toujours par la combinaison "loi de Laplace + compression devenant excessive sur surface à faible rayon de courbure".

- surtout, en cas d'insuffisance artérielle sévère (indice de pression à la cheville < 0,50), d'artériopathie très distale, de peaux vulnérables (diabétiques avec microangiopathie évoluée, patients sous corticothérapie au long cours, sujets très âgés avec atrophie cutanée, ...), le risque iatrogène d'une pression trop élevée sur le cou de pied et le dos du pied devient très sensible. La contention ne peut dans ces cas là relever que d'un avis spécialisé.

Au total, le seul intérêt de la compression au niveau du pied est de :

- prévenir un oedème et une stase veineuse au niveau du coude pied, en raison de la transition de pression trop brusque du pied vers la cheville (où s'exerce la pression la plus forte),
- faire tenir le bas correctement en place.

De plus, la pression n'est pas contrôlée au niveau du pied et par conséquent elle peut même être au niveau du cou de pied supérieure à celle exercée au niveau de la cheville.

L'un des buts de l'invention est de proposer une orthèse de contention médicale permettant de remédier à ces inconvénients grâce à un nouveau profil de pression défini spécifiquement pour la partie de pied.

Plus précisément, l'invention vise une orthèse de contention médicale présentant un profil de pression où la pression délivrée au niveau du cou de pied est ramenée à une valeur proche de celle procurée par un textile non médical. Au niveau de la zone malléolaire, la pression est délivrée à une valeur médiane entre celle délivrée au niveau du cou de pied et la valeur prescrite au niveau de la cheville en fonction de la classe de contention choisie. De cette manière, l'orthèse présente une progressivité du profil de pression entre le pied et la cheville, permettant de se prémunir d'un effet garrot au niveau de la cheville.

L'orthèse de l'invention a la forme d'un article tricoté de type bas, chaussette ou collant, comportant une partie de jambe et une partie de pied. La partie de jambe, qui s'étend vers le haut à partir de la cheville, est une partie tubulaire compressive dont la structure de maille et le dimensionnement sont choisis de manière à appliquer sur la jambe une pression de contention thérapeutique une fois l'orthèse enfilée sur le membre. La partie de pied, qui s'étend à partir de la région malléolaire jusqu'à la région des orteils en couvrant la région du cou de pied, est une partie conformée dont la structure de maille et le dimensionnement sont choisis de manière à envelopper élastiquement le pied une fois l'orthèse enfilée sur le membre.

De façon caractéristique de l'invention, la structure de maille et le dimensionnement de la partie de pied sont choisis de manière à vérifier les relations (i) $0 < P_x < P_m < P_b$ et (ii) $P_m < 36$ mmHg une fois l'orthèse enfilée sur le membre, $P_x$ étant la pression appliquée au niveau du cou de pied, $P_m$ étant la pression appliquée au niveau des malléoles, et $P_b$ étant la pression de contention thérapeutique appliquée au niveau de la cheville.

On notera que la relation $P_m < 36$ mmHg est une condition spécifiant que l'orthèse est destinée à traiter l'insuffisance veineuse chronique et ne relève pas d'un autre d'un domaine thérapeutique.

De préférence, la structure de maille et le dimensionnement de la partie de pied sont choisis tels que $P_m < 0,8.P_b$ et/ou pour que la pression $P_x$ au cou de pied s'établisse à 5 mmHg $\pm$ 25 % et/ou pour que la pression $P_m$ exercée sur la zone rétromalléolaire s'établisse à une valeur intermédiaire entre celles exercées sur le cou de pied et sur la cheville, c'est-à-dire avec $P_m = 0,5.(P_b + P_x) \pm 25$ %.

La pression au niveau du cou de pied peut être évaluée par une moyenne des pressions prises sur le dessus, la face interne et la face externe de la région du cou de pied. La pression au niveau des malléoles peut être évaluée par une moyenne des pressions prises sur la face antérieure, la face postérieure, la face interne et la face externe de la région malléolaire.

On va maintenant décrire un exemple de mise en oeuvre de l'invention, en référence aux dessins annexés où les mêmes références désignent d'une figure à l'autre des éléments identiques ou fonctionnellement semblables.

La figure 1 est une vue schématique illustrant les différentes zones concernées par le profil de pression de l'orthèse de l'invention.

La figure 2 montre les divers points de mesure qui peuvent être utilisés pour le recueil et le calcul des données de pression.

La figure 3 illustre les pressions exercées respectivement au niveau du cou de pied, de la malléole et de la cheville, pour des orthèses de classe I, II et II selon l'invention.

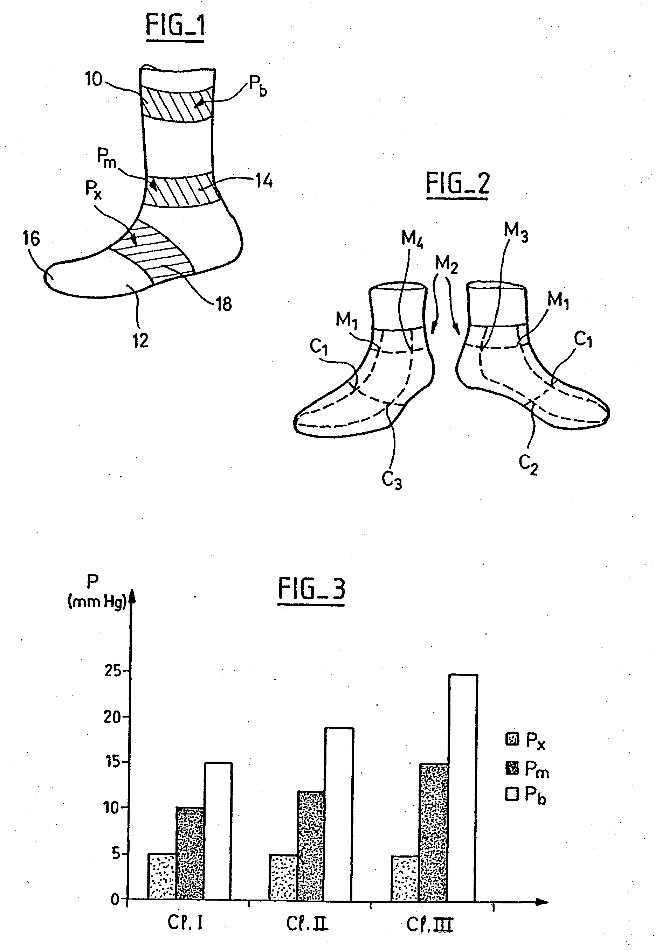

Sur la figure 1, on a représenté les diverses régions de la partie inférieure de l'orthèse, qui est une orthèse fermée en partie inférieure, c'est-à-dire qui enveloppe le pied, par exemple une chaussette, un bas ou un collant.

Ces différentes régions comprennent une partie de jambe 10 s'étendant vers le haut à partir de la cheville (et incluant cette région), et une partie de pied 12 s'étendant à partir de la région malléolaire 14 (et incluant cette région) jusqu'à la région 16 des orteils, en couvrant la région intermédiaire du cou de pied 18.

La partie de jambe 10 est une partie tubulaire compressive, en elle-même connue et non modifiée, dont la structure de maille et le dimensionnement sont choisis de manière à appliquer sur la jambe une pression de contention thérapeutique une fois l'orthèse enfilée sur le membre.

La maille peut être du même type que celle des bas de contention classiques, par exemple de type tramée, jersey, micromesh pincée ou flottée, etc., toutes mailles connues en elles-mêmes du spécialiste des techniques de tricotage. En ce qui concerne le fil choisi pour le tricotage de cette orthèse, celui-ci peut être un élasthanne guipé coton et polyamide, un élasthanne guipé polyamide sans coton, ou encore un mélange d'élasthanne et d'élasto-diène (latex de caoutchouc synthétique).

Le choix de la maille et des fils, ainsi que le dimensionnement des différentes rangées de maille, sont définis de manière à appliquer à différentes altitudes de la jambe des pressions prédéterminées, par exemple à la hauteur de la cheville, au départ du mollet, au niveau du mollet, au poplité, etc. jusqu'en haut de cuisse dans le cas d'un bas-cuisse ou d'un collant. Ces différentes pressions sont définies pour chaque classe de contention en référence à des gabarits métrologiques tels que le modèle Hohenstein : pour une altitude donnée, la structure de la rangée de mailles assure une répartition homogène des forces de rappel élastique sur la circonférence du bas, c'est-à-dire le long d'un contour correspondant à une section horizontale du membre. Dans le cas d'un contour circulaire, ce qui est le cas des modèles Hohenstein, l'application de ces forces de rappel élastique sur le périmètre du contour circulaire correspondant engendre en un point donné, selon la loi de Laplace, une pression locale inversement proportionnelle au rayon de courbure du contour en ce point.

Dans le cas présent, on considèrera essentiellement la pression exercée à l'altitude dite "du point *b*" du modèle Hohenstein, c'est-à-dire au niveau de la cheville, qui est la pression prescrite pour la classe de contention choisie (I, II, III ou IV), et qui par ailleurs correspond à la valeur de pression (théoriquement) la plus élevée exercée sur le membre. La partie de pied, quant à elle, est une partie conformée, c'est-à-dire destinée à épouser la forme du pied, et dont la structure de maille et le dimensionnement sont choisis de manière à envelopper élastiquement le pied une fois l'orthèse enfilée sur le membre, de manière à permettre un maintien satisfaisant de la partie de jambe, sans formation de plis ni déplacement de la matière de l'orthèse par rapport au pied pendant la marche.

Compte tenu de la morphologie du pied et de la présence osseuse dans les régions malléolaires 14 et du cou de pied 18, la compression n'est pas uniforme sur tout le contour. On définit alors la pression $P_m$ appliquée au niveau des malléoles et la pression $P_x$ appliquée au niveau du cou de pied à partir de moyennes des pressions locales relevées en plusieurs points. La figure 2 illustre un exemple de points de mesure pour l'évaluation de ces valeurs de pression.

Pour la région malléolaire, la pression peut être relevée en quatre points répartis de la manière suivante : $M_1$ sur la face antérieure, $M_2$ sur la face postérieure, $M_3$ sur la face interne et $M_4$ sur la face externe, juste en dessous des malléoles. Pour la région du cou de pied, ces points peuvent être répartis de la manière suivante : $C_1$ sur le dessus du pied, $C_2$ sur la face interne et $C_3$ sur la face externe. Le paramètre de pression $P_m$ est la moyenne des quatre valeurs $M_1$ - $M_4$ ainsi relevées ; de même la pression $P_x$ est la moyenne des trois valeurs $C_1$ - $C_3$ ainsi relevées.

Ces pressions peuvent être relevées au moyen de capteurs en eux-mêmes classiques (capteurs de type Salzmann ou autres) qui mesurent la pression produite localement par l'orthèse en délivrant un signal électrique qui pourra ensuite être converti et traité pour divers calculs et affichages. L'invention propose un nouveau profil de pression exercé au niveau du pied par l'orthèse de contention (les profils au niveau de la partie de jambe n'étant pas modifiés)

L'orthèse est ainsi conçue pour délivrer une pression au niveau du cou de pied par exemple de 5 mmHg, soit une valeur proche d'un textile non médical, suffisante pour permettre un enveloppement du pied et un maintien de l'orthèse sans gêne pour le sujet.

Au niveau de la région malléolaire, l'orthèse est modifiée pour produire à cet endroit une valeur de pression $P_m$ qui soit intermédiaire, avantageusement médiane, entre la pression $P_x$ = 5 mmHg exercée au niveau du cou de pied et la valeur $P_b$ exigée au point b (cette dernière valeur dépendant de la classe de contention choisie). Ainsi, l'orthèse est par exemple apte à délivrer dans la région malléolaire une pression :

- $P_m$ = 7,5-10 mmHg pour un produit de classe I où $P_b$ = 10-15 mmHg),
- $P_m$ = 10-12,5 mmHg pour un produit de classe II (où $P_b$ = 15-20 mmHg),
- $P_m$ = 12,5-20,5 mmHg pour un produit de classe III (où Pb = 20-36 mmHg),
- $P_m$ < 36 mmHg pour un produit de classe IV (où Pb > 36 mmHg),

Cette progressivité, exprimée par la relation

$$0 < P_x < P_m < P_b,$$

permet de se prémunir d'un effet garrot au niveau du point b.

La figure 3 illustre ainsi des exemples de pressions $P_x$, $P_m$ et $P_b$ pour des orthèses de classe I, II et II selon l'invention.

Au-delà du point b, le produit reste conforme aux prescriptions réglementaires et, quelle que soit la classe, ne nécessite donc pas une nouvelle certification par rapport au produit existant.

Les faibles pressions au niveau des pieds sont susceptibles d'améliorer notablement l'observance de la contention veineuse élastique, et donc l'efficacité thérapeutique à long terme du traitement.

**Revendications**

1. Une orthèse compressive de contention du membre inférieur de classe I, II, III ou IV en forme d'article tricoté de type bas, chaussette ou collant, comportant :

   - une partie de jambe (10) s'étendant vers le haut a partir de la cheville, cette partie de jambe étant une partie tubulaire compressive dont la structure de maille et le dimensionnement sont choisis de manière à appliquer sur la jambe une pression de contention thérapeutique une fois l'orthèse enfilée sur le membre, et
   - une partie de pied (12), s'étendant a partir de la région malléolaire (14) jusqu'à la région des orteils (16) en couvrant la région du cou de pied (18), cette partie de pied étant une partie conformée dont la structure de maille et le dimensionnement sont choisis de manière à envelopper élastiquement le pied une fois l'orthèse enfilée sur le membre,

   orthèse **caractérisée en ce que** la structure de maille et le dimensionnement de la partie de pied sont choisis de manière que, une fois l'orthèse enfilée sur le membre :

   $$0 < P_x < P_m < P_b$$

   et

   $$P_m < 36 \text{ mmHg}$$

   $P_x$ étant la pression appliquée par l'orthèse au niveau du cou de pied,
   $P_m$ étant la pression appliquée par l'orthèse au niveau des malléoles, et
   $P_b$ étant ladite pression de contention thérapeutique appliquée par l'orthèse au niveau de la cheville, lesdites pressions étant définies en référence à un modèle Hohenstein de gabarit métrologique.

2. L'orthèse de la revendication 1, où la structure de maille et le dimensionnement de la partie de pied sont choisis de manière que $P_m < 0,8 \, P_b$.

3. L'orthèse de la revendication 1, où la structure de maille et le dimensionnement de la partie de pied sont choisis de manière que $P_x = 5$ mmHg $\pm 25$ %

4. L'orthèse de la revendication 1, où la structure de maille et le dimensionnement de la partie de pied sont choisis de manière que $P_m = 0,5.(P_b + P_x) \pm 25$ %.

5. L'orthèse de la revendication 1, où ladite pression appliquée par l'orthèse au niveau du cou de pied est une moyenne des pressions prises sur le dessus (C1), la face interne (C2) et la face externe (C3) de la région du cou de pied.

6. L'orthèse de la revendication 1, où ladite pression appliquée par l'orthèse au niveau des malléoles est une moyenne des pressions prises sur la face antérieure (M1), la face postérieure (M2), la face interne (M3) et la face externe (M4) de la région malléolaire.

**Patentansprüche**

1. Kompressionsorthese der Klasse I, II, III oder IV zum Stützen der unteren Gliedmaße in Form eines gestrickten Artikels von der Art Strumpf, Socke oder Strumpfhose, die aufweist:

   - einen Beinteil (10), der sich ausgehend vom Fußknöchel nach oben erstreckt, wobei dieser Beinteil ein rohrförmiger Kompressionsteil ist, dessen Maschenstruktur und Abmessung so gewählt werden, dass ein therapeutischer Stützdruck auf das Bein ausgeübt wird, wenn die Orthese auf die Gliedmaße aufgestreift wurde, und
   - einen Fußteil (12), der sich vom Knöchelbereich (14) bis zum Bereich der Zehen (16) erstreckt, indem er den Bereich des Spanns (18) bedeckt; wobei dieser Fußteil ein angepasster Teil ist, dessen Maschenstruktur und Abmessung so gewählt werden, dass der Fuß elastisch umhüllt wird, wenn die Orthese auf die Gliedmaße aufgestreift wurde,

   wobei die Orthese **dadurch gekennzeichnet ist, dass** die Maschenstruktur und die Abmessung des Fußteils so gewählt werden, dass, wenn die Orthese auf die Gliedmaße aufgestreift wurde, gilt:

   $$0 < P_x < P_m < P_b$$

   und

$$P_m < 36 \ mmHg$$

wobei

> $P_x$ der von der Orthese im Bereich des Spanns ausgeübte Druck ist,
> $P_m$ der von der Orthese im Bereich der Knöchel ausgeübte Druck ist, und
> $P_b$ der therapeutische Stützdruck ist, der von der Orthese im Bereich des Fußknöchels ausgeübt wird, wobei die Drücke bezüglich eines Hohenstein-Modells einer messtechnischen Lehre definiert werden.

**2.** Orthese nach Anspruch 1, wobei die Maschenstruktur und die Abmessung des Fußteils so gewählt werden, dass gilt $P_m < 0{,}8 \ P_b$.

**3.** Orthese nach Anspruch 1, wobei die Maschenstruktur und die Abmessung des Fußteils so gewählt werden, dass gilt $P_x = 5 \ mmHg \pm 25 \ \%$.

**4.** Orthese nach Anspruch 1, wobei die Maschenstruktur und die Abmessung des Fußteils so gewählt werden, dass gilt $P_m = 0{,}5.(P_b + P_x) \pm 25 \ \%$.

**5.** Orthese nach Anspruch 1, wobei der von der Orthese im Bereich des Spanns ausgeübte Druck ein Mittelwert der Drücke ist, die an der Oberseite (C1), der Innenseite (C2) und der Außenseite (C3) des Spannbereichs des Fußes gemessen werden.

**6.** Orthese nach Anspruch 1, wobei der von der Orthese im Bereich der Knöchel ausgeübte Druck ein Mittelwert der Drücke ist, die an der Vorderseite (M1), der Rückseite (M2), der Innenseite (M3) und der Außenseite (M4) des Knöchelbereichs gemessen werden.

**Claims**

**1.** A compressive orthosis for lower limb splinting of class I, II, III or IV, in the form of a knitted item of the sock, stocking or pair of tights type, including:

> - a leg part (10) extending upward from the ankle, said leg part being a compressive tubular part whose stitch structure and size are chosen so as to apply on the leg a therapeutic splinting pressure once the orthosis slipped on the limb, and
> - a foot part (12) extending from the malleolar region (14) to the toe region (16), covering the instep region (18), said foot part being a conformed part whose stitch structure and size are chosen so as to elastically envelop the foot once the orthosis slipped on the limb,

the orthosis being **characterized in that** the stitch structure and the size of the foot part are chosen so that, once the orthosis slipped on the limb:

$$0 < P_x < P_m < P_b$$

and

$$P_m < 36 \ mmHg,$$

> $P_x$ being the pressure applied by the orthosis at the instep,
> $P_m$ being the pressure applied by the orthosis at the malleolus, and
> $P_b$ being said therapeutic splinting pressure applied by the orthosis at the ankle,

said pressures being defined with reference to a Hohenstein model of metrological template.

**2.** The orthosis of claim 1, wherein the stitch structure and the size of the foot part are chosen so that $P_m < 0.8 \cdot P_b$.

**3.** The orthosis of claim 1, wherein the stitch structure and the size of the foot part are chosen so that $P_x = 5 \ mmHg \pm 25 \ \%$.

**4.** The orthosis of claim 1, wherein the stitch structure and the size of the foot part are chosen so that $P_m = 0.5 \cdot (P_b + P_x) \pm 25 \ \%$.

**5.** The orthosis of claim 1, wherein said pressure applied by the orthosis at the instep is an average of the pressures taken on the top (C1), the inner face (C2) and the outer face (C3) of the instep region.

**6.** The orthosis of claim 1, wherein said pressure applied by the orthosis at the malleolus is an average of the pressures taken on the front face (M1), the rear face (M2), the inner face (M3) and the outer face (M3) of the malleolar region.

FIG_1

FIG_2

FIG_3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 3856008 A
- EP 0934043 B1